# EUROPEAN PATENT SPECIFICATION

(11) **EP 4 213 786 B1**
(45) Date of publication and mention of the grant of the patent: **29.04.2026**
(21) Application number: 21798243.8
(22) Date of filing: 21.09.2021
(51) Int. Cl.: A61F 13/68, A61F 13/505, A61F 13/49

(54) **ADJUSTABLE OUTER COVER FOR TWO PIECE ABSORBENT ARTICLE**
VERSTELLBARE ÄUSSERE ABDECKUNG FÜR ZWEITEILIGEN SAUGFÄHIGEN ARTIKEL
COUVERTURE EXTÉRIEURE RÉGLABLE POUR ARTICLE ABSORBANT EN DEUX PARTIES

(30) Priority: 21.09.2020 US 202063080868 P
(43) Date of publication of application: 26.07.2023
(73) Proprietor: The Procter & Gamble Company, Cincinnati, OH 45202 (US)
(72) Inventor: ROE, Donald, Carroll, Cincinnati, Ohio 45202 (US); SCHOENBORN, Udo, Friedel, 65824 Schwalbach am Taunus (DE); CHEN, Yin, 65824 Schwalbach am Taunus (DE); GIOVANNI, Sara, Lyn, Cincinnati, Ohio 45202 (US); WEST, Tonya, Mae Sheldon, Cincinnati, Ohio 45202 (US); WIZENBERG, Gaëlle, Sandra, Sarasota, Florida 34231 (US); MONTILLA RAMOS, Ana Cecilia, 65824 Schwalbach am Taunus (DE)
(74) Representative: P&G Patent Germany
(86) International application number: PCT/US2021/071527
(87) International publication number: WO 2022/061374

(56) References cited:
- EP-A1- 2 717 817
- WO-A1-97/25951
- US-A- 5 368 585
- US-A1- 2008 195 075
- US-A1- 2014 221 954
- US-A1- 2014 296 816

## Description

### FIELD

The present disclosure relates generally to the field of wearable absorbent articles having absorbent inserts and reusable outer covers, and more particularly, systems for adjusting the fit of such absorbent articles.

### BACKGROUND

It has long been known that absorbent articles (e.g., diapers, adult incontinence articles, feminine hygiene pads) offer the benefit of receiving and containing urine and/or other bodily exudates (e.g., feces, menses, mixture of feces and urine, mixture of menses and urine, etc.). It has been proposed to manufacture two-piece absorbent articles with a reusable outer cover and a detachable absorbent insert that may be reusable or disposable. In this way, the insert may be made with materials known to provide even more superior performance while minimizing the amount of waste as the whole article need not be disposed.

To effectively contain bodily exudates, the article should provide a snug fit around the waist and legs of a wearer. Fastening systems and leg elastics have been used to ensure the article is secured about the wearer and remains in place. However, such systems are typically limited in their ability to secure an article about differently sized wearers, resulting in product lines of different sizes and/or inadequate sizing mechanisms. Consumers, particularly consumers of reusable outer covers (which tend to be formed of more expensive and/or more durable materials), desire longer lasting articles that may be adjusted to fit the wearer as they grow. In addition, for some wearers, articles fit well about the waist but not about the legs or vice versa, or fit well about one leg but not about the other, resulting in the wearer be unable to obtain a proper fit regardless of the article's size. Further, some known adjustment systems are hidden so as to not irritate the skin during wear or undermine aesthetics. Yet, such configurations prevent the user from readily identifying the mechanism, which can result in a failure to use it and ultimate dissatisfaction with the product.

US5368585A is concerned with a diaper comprising a gas permeable, water impervious outer cover and a water absorbent reusable pad. The pad and cover have mating hook and loop fasteners to secure the pad to the cover. The cover has fastening means, in particular an extended belt portion, for connecting the cover around the waist of the wearer.

WO97/25951A1 relates to an absorbent article having an adjustment system on the backsheet and/or the topsheet to be adjusted to securely fit the absorbent article about a wearer's waist and legs. The adjustment system comprises an adjustment tab with a fixed portion secured to the article, an intermediate portion and grasping portion.

US2014/296816A1 discloses absorbent articles having a pair of leg opening adjustment mechanisms. These can be independently adjusted to provide a desired tension and/or closure of the absorbent article about the legs of the wearer of the absorbent article.

EP2717817A1 relates to a diaper sheath having a shell with an inner panel and an outer panel. The diaper sheath includes a first intermediate pocket and a second intermediate pocket each having at least one opening. The diaper sheath further includes a first intermediate strip and a second intermediate strip set inside each of the first and second intermediate pockets respectively. The intermediate strips can be adjusted in length to securely fit the absorbent article about a wearer's waist and legs.

Therefore, there is a need for improved size adjustment mechanisms. Further, there is a need for mechanisms that adjust one area of an article independently of other areas. There is also a need to provide means for users to quickly locate and/or understand how to best utilize the adjustment system.

### SUMMARY

The invention provides a diaper outer cover comprising an adjustment system as defined in the claims.

### BRIEF DESCRIPTION OF THE DRAWINGS

Fig. 1 is a perspective view of a wearable absorbent article as it might appear being worn by a wearer about the lower torso, according to a nonlimiting embodiment of the present invention;
Fig. 2 is a plan view of an outer cover opened and laid flat, wearer-facing surface facing the viewer, according to a nonlimiting embodiment of the present invention;
Fig. 3 is a plan view of an absorbent insert opened and laid flat, garment-facing surface facing the viewer, according to a nonlimiting embodiment of the present invention;
Fig. 4 is a plan view of a leg band portion and partial waist region according to a nonlimiting embodiment of the present invention;
Fig. 5 is a plan view of an adjustment strip according to a nonlimiting embodiment of the present invention with both the unadjusted and the adjusted configurations shown;
Fig. 5A is a plan view of an adjustment strip according to a nonlimiting embodiment of the present invention;
Figs. 5B-5C are plan views of exemplary adjustment systems according to nonlimiting embodiments of the present invention with both the unadjusted and the adjusted configurations shown;
Figs. 6-7 are plan views of exemplary adjustment systems according to nonlimiting embodiments of the present invention with both the unadjusted and the adjusted configurations shown;
Fig. 8A is a plan view of an exemplary adjustment system according to a nonlimiting embodiment of the present invention with both the unadjusted and the adjusted configurations shown;
Fig. 8B is a side elevation view the exemplary adjustment system of Fig. 8A with both the unadjusted and the adjusted configurations shown;
Fig. 8C is a plan view of an exemplary adjustment system according to a nonlimiting embodiment of the present invention with both the unadjusted and the adjusted configurations shown;
Fig. 8D is an exemplary locking clip mechanism with both a plan view and a perspective view shown;
Figs. 9-11 are plan views of exemplary adjustment systems according to nonlimiting embodiments of the present invention with both the unadjusted and the adjusted configurations shown;
Fig. 12 is a plan view of an outer cover opened and laid flat, garment-facing surface facing the viewer, with adjustment system openings disposed in the garment-facing surface;
Fig. 13 is a plan view of an outer cover opened and laid flat, wearer-facing surface facing the viewer, with adjustment system openings disposed in the wearer-facing surface;
Fig. 14 is a plan view of an outer cover opened and laid flat, wearer-facing surface facing the viewer, with adjustment system openings disposed at the junction of the first material and the second material;
Fig. 15 is a perspective view of an exemplary guide in the form of an x-buckle;
Fig. 16 is a perspective view of an exemplary guide in the form of a set of double rings; and
Figs. 17A-17B are cross sectional views of exemplary of absorbent insert.

### DETAILED DESCRIPTION

"Absorbent article" means a device that absorbs and contains body exudates and, more specifically, devices that are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Absorbent insert" and "insert" mean a component of a wearable absorbent article that is adapted to contain and/or absorb urine, feces, menses or any combination thereof, and is adapted to be installable and removable as a modular unit from an outer cover. Herein, an absorbent insert may also be referred to as an "absorbent assembly". The terms "absorbent insert," "insert" and "absorbent assembly" may be used interchangeably herein.

"Disposed" refers to an element being located in a particular place or position. A feature that is disposed on a surface or side of a component may be integral with said component or may be joined to said component.

"Disposable", when referring to an absorbent insert, means that the absorbent insert is not adapted or intended to be effectively sanitarily laundered in an ordinary household laundering process and ordinary household equipment, and thereby is ordinarily unsuitable for sanitary and effective reuse so as to provide as-new intended functions and performance, following soiling by exudates and removal from an outer cover. By way of non-limiting examples, effective laundering may be frustrated or prevented, causing the insert to be disposable, by inclusion of materials and/or construction: that do not retain their substantial as-new physical shape or structure through ordinary household laundering and drying so as to be effective as-new in reuse; that absorb aqueous liquids and cannot be sufficiently dried/dehydrated in ordinary household drying equipment and ordinary drying cycles so as to be effective as-new in reuse; that dissolve or substantially degrade in ordinary household laundering or drying, causing the insert to be substantially damaged or rendered useless; and/or that cannot be effectively cleaned of exudate material through ordinary laundering, so as to be sanitary and otherwise acceptable for re-use.

"Inboard," with respect to a first feature of an article and its position relative a second feature or location on the article, means that the first feature lies closer to a respective axis of the article than the second feature or location, along a horizontal x-y plane approximately occupied by the article when laid out flat, extended to the full longitudinal and lateral dimensions of its component web materials against any contraction induced by any included pre-strained elastomeric material, on a horizontal surface. Laterally inboard means the first feature is closer to the longitudinal axis, and longitudinally inboard means the first feature is closer to the lateral axis. Conversely, "outboard," with respect to a first feature of an article and its position relative a second feature or location on the article, means that the first feature lies farther from the respective axis of the article than the second feature or location.

"Integral" means configurations whereby an element is created from or created by an article component, or portions thereof, as opposed to being joined to the component. "Integrally formed" means an element is created from an underlying material or portion thereof, by for example molding, shaping and/or reconstituting the material.

"Joined" or "attached" means configurations whereby an element is directly secured to another element by affixing the element directly to the other element, and configurations whereby an element is indirectly secured to another element by affixing the element to intermediate member(s) that in turn are affixed to the other element.

"Longitudinal" means a direction lengthwise in a component such that the longitudinal direction runs parallel to the maximum linear dimension in the x-y plane of the component. In an absorbent article as described herein, the longitudinal direction runs substantially perpendicular from a waist end edge to an opposing waist end edge when the absorbent article is in a flat out, uncontracted state, or from a waist end edge to the bottom of the crotch in a bifolded article.

"Lateral" refers to a direction generally perpendicular to the longitudinal direction. **In** the absorbent article described herein, the lateral direction runs substantially parallel from a side edge to an opposing side edge.

"Outer cover" means a component of a wearable absorbent article that is adapted to be worn about the lower torso of a wearer, and is adapted to support an absorbent insert and hold the insert close to the wearer's body.

"Reusable", when referring to a component means that the component is adapted to be used for its intended purpose after initial use without substantial destruction of any portions of the component necessary for as-new functionality. For example, a reusable outer cover means an outer cover that is adapted to permit removal of at least a first insert, and replacement thereof with at least a second insert, without substantial destruction of any portions of the outer cover that are necessary to provide the substantial as-new functionality of the outer cover, and without the necessity of any repair or reconstruction following such insert replacement.

### Overview

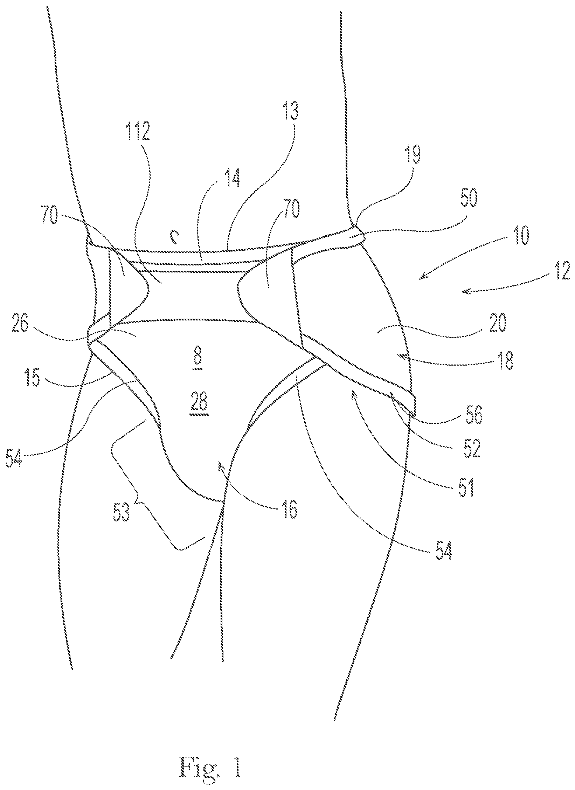

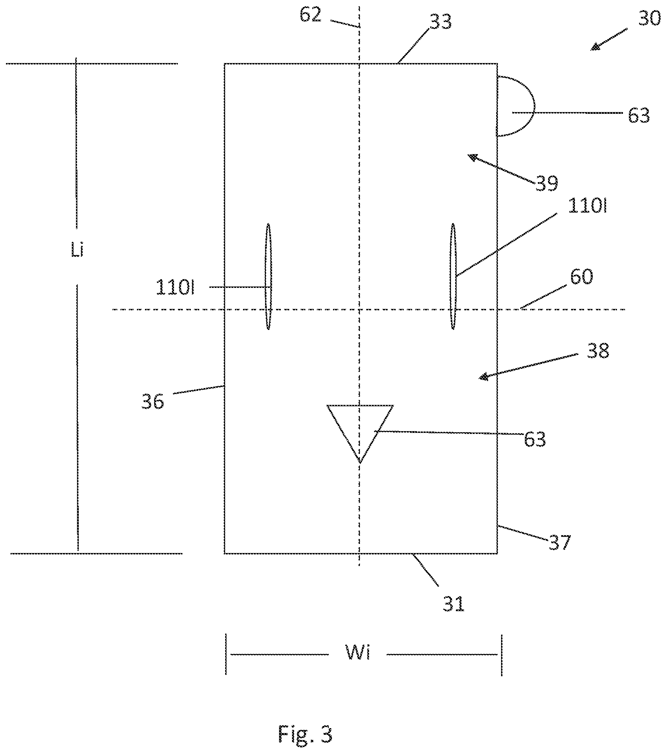

Fig. 1 is a perspective view an exemplary, nonlimiting embodiment of an absorbent article 10. The article is shown in the form of a two-piece absorbent article 12, having an outer cover 20 and an absorbent insert 30 shown in Fig. 3. It is to be understood that during manufacturing, the article may comprise several discrete pieces that are joined together. However, by two-piece absorbent article, it is meant that the article in its final form has two components that the user assembles together for wear. While shown as a two-piece absorbent article, it is also contemplated that the article may comprise more than two pieces, such as attachable ears or belts. Returning to Fig. 1, it can be seen that the absorbent article 10 may have a front waist region 14, a rear waist region 18 and a crotch region 16 disposed between the front and rear regions. The article may be placed on a wearer by wrapping the outer cover 20 between the wearer's legs and under the buttocks such that the crotch region 16 is between the wearer's legs. When the insert 30 has been installed into or onto the outer cover 20, the insert 30 will then be disposed proximate to the wearer. Nonlimiting examples of two-piece articles are disclosed in U.S. Pat. Nos. 8,998,870, 9,089,456, 8,435,223, 9,011,402, 8,808,263, 8,759,605 and 8,932,273 and 9,078,789. In various embodiments, the outer cover is reusable.

The outer cover comprises one or more adjustment strips 40 that each can be used to adjust a leg gasketing system 52 to achieve a more secure fit about the wearer. The initial maximum dimension is reduced and/or more tension is created in the adjustment strip through adjusting the strip by the means discussed herein.

### Outer Cover

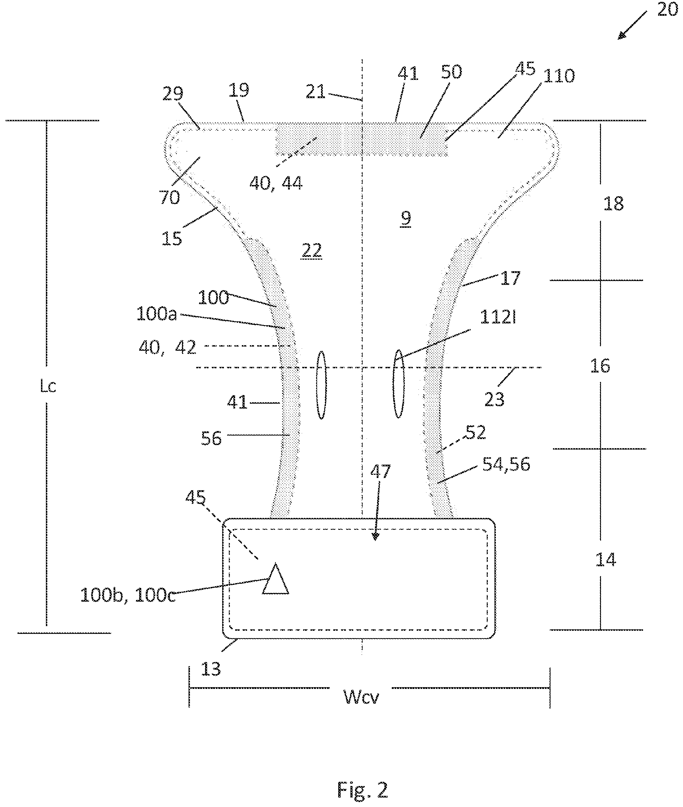

Fig. 1 depicts an exemplary outer cover, with its garment-facing surface 8 facing the viewer, and Fig. 2 depicts an exemplary outer cover 20 as it may appear opened and laid substantially with the wearer-facing surface 9 facing the viewer. The outer cover comprises a front lateral edge 13, a rear lateral edge 19, a first longitudinal edge 15 and a second longitudinal edge 17. The outer cover comprises a length, Lc, from the outboard-most portion of the front lateral edge 13 to the outboard-most portion of the rear lateral edge 19. The outer cover may comprise a lateral axis 23, dividing evenly its length, and a longitudinal axis 21. The longitudinal edges 15, 17 may be parallel to the longitudinal axis 21. However, for better fit, longitudinal edges 15, 17 may be curved or angled to produce, for example, an "hourglass" shape article when viewed in a plan view as shown in Fig. 2, for example. In nonlimiting examples, the outer cover may be asymmetric, for instance having a width in its rear region that is greater than the width in its front region. Thus, the width of the outer cover, Wcv, as measured between the longitudinal edges along a line parallel to the lateral axis, may vary along the longitudinal length of the outer cover. Nonlimiting examples of outer covers are disclosed in U.S. Pat. Nos. 9,387,138 and 8,435,223.

The outer cover, and article, comprises a front waist region 14, a rear waist region 18 and a crotch region 16 between the first and second waist regions.

Outer cover 20 and/or layers or portions thereof may be made of any durable or semi-durable knitted, woven or nonwoven textile or textile-like material that is appropriately compatible with skin of the intended wearer(s). Outer cover 20 may be formed of a single layer of a durable or semi-durable material, or may have two or more layers in the front waist region 14 and/or rear waist region 18. Suitable examples are described in U.S. Applications Ser. Nos. 12/687,493; 12/687,412; 12/687,528; and 12/687,425 (all by Roe et al.). Non-limiting examples of fibers, nonwovens and laminates of nonwovens and films that might be considered for use as semi-durable outer cover materials may be found in U.S. Patents Nos. 7,223,818; 7,211,531; 7,060,149; 6,964,720; 6,905,987; 6,890,872; 6,884,494; 6,878,647; and 5,518,801; and U.S. Published Applications Nos. 2008/0319407; 2008/0045917; 2007/0293111; 2007/0287983; 2007/0287348; 2007/0249254; 2007/0203301; and 2005/0164587.

The outer cover further comprises a first exterior surface 22 and a second exterior surface 28, which opposes the first exterior surface. One exterior surface will serve as the wearer-facing surface 9, and the other will serve as the garment-facing surface 8. In some embodiments, the exterior surfaces may be formed from the same materials. However, due to different functional needs of the garment-facing surface and the wearer-facing surfaces, it may be desirable that the exterior surfaces comprise different materials.

In nonlimiting examples, the wearer-facing surface of the outer cover may be formed from a first material or first combination of materials 24 (collectively referred to as "first material" hereinafter). The material selected may include hydrophilic fibers, or fibers treated to be hydrophilic. This may be desired in some circumstances to cause the material forming wearer-facing surface 9 to more readily absorb liquid, or transmit liquid therethrough. This may serve to provide supplemental absorbency within the outer cover for an event in which liquid exudates escape the insert, reducing the likelihood that the outer cover will leak. Alternatively, it may provide one way of communicating to the user that liquid exudates have escaped the insert, by causing wetness to be transmitted through to the outer cover such that wetness is sensed by the wearer and/or visible on exterior surfaces. Alternatively, it may serve to provide a layer that tends to draw moisture away from the skin, for a drier, more comfortable feel. Additionally, or alternatively, it may be desirable to form the wearer-facing surface from an apertured material to facilitate transfer of exudates through the wear-facing surface and onto the absorbent insert. In some nonlimiting examples, the material may include fibers having hydrophobic properties, providing enhanced liquid containment attributes to the wearer-facing layer.

The garment-facing surface of the outer cover may be formed by a second material or a second combination of materials 26 (collectively referred to as "second material" hereinafter). The material selected may include fibers having hydrophobic properties, providing enhanced liquid containment attributes to such layer or may include a liquid impermeable material. In another example, however, it may be desirable in some circumstances for the selected material to include hydrophilic fibers, or fibers treated to be hydrophilic, so as will cause the material to more readily absorb liquid. This may serve to provide supplemental absorbency within the outer cover for the event in which liquid exudates escape the insert, or to provide one way of communicating to the user that liquid exudates have escaped the insert. Additionally, in some circumstances, it may be desirable that the material selected have soft tactile properties so as to have a pleasant feel that the user and/or wearer find attractive. The material also may be selected so as to have a desired appearance, including but not limited to coloration, sheen, and/or texture.

The first material 24 and the second material 26 may differ by one of the group consisting of: of texture, caliper, elasticity, opacity, water resistance, absorbency, breathability and combinations thereof.

In nonlimiting examples, the outer cover may be reversible such that both the first exterior surface and the second exterior surface are adapted to function as the wearer-facing surface and the garment-facing surface. This may provide, for example, variation in visible graphics or colors, textures and/or breathability against skin, liquid wicking properties and the like for the wearer.

Layers or other elements of the outer cover may be joined to each other via any suitable mechanism, including, for example, adhesives, mechanical bonding, ultrasonic bonding, sewing, stitching, serging, edging, and the like. In nonlimiting examples, the layers forming the first and second exterior surfaces may be joined together by a seam 29 as illustrated in Fig. 2, which may join the materials proximate to the first and second lateral edges and/or about the first and second longitudinal edges. The seam may be continuous. The seam may be continuous except where the opening(s) 45 exist, except where flap 47 openings exist, except where leg bands 56 are present and/or except where a waist feature 50 exists. In nonlimiting examples, the seam may be used to form a portion of the leg band 56 and/or to form a portion of a waist feature. In some nonlimiting examples, the seam may be inboard the edge of one or both materials as shown in Fig. 2. In such examples, a second seam may be present along the edge to further secure the materials together. The seam may be formed through any suitable means, including adhesive, pressure bonding, heat bonding, stitches and combinations thereof.

### Waist Features, Leg Gasketing Systems, Adjustment Systems

Referring again to Figs. 1-2, the article and outer cover may comprise one or more waist features 50. The waist feature may be elastic and thereby provide better fit about the waist of the wearer. Elasticized waist features include waistbands, waist cuffs having pockets formed from a portion of the waist feature that is unattached from the remaining portions of the outer cover, and waist panels and/or belts designed to fit securely about the abdomen of the wearer. Nonlimiting examples of elasticized waist features are disclosed in U.S. Patent App. Nos. 14/533,472; 15/074,675 and 62/855,001. Elasticized waist features may comprise one or more nonwoven or textile layers, which may be layers of the outer cover or discrete portions, and one or more elastic elements. In nonlimiting examples, the elasticized waist feature comprises elastic strands or ribbons joined to the nonwoven and/or textile layer(s). In further nonlimiting examples, the elasticized waist feature comprises a laminate of one or more textile and/or nonwoven layers and one or more films. The elasticized waist element may comprise one or more rugosities if the elastic material is strained prior to lamination. In various embodiments, the elastic elements may be affixed within the outer cover only at or near their respective ends, e.g., within a pouch, tube or envelope structure formed of outer cover material - referred to herein as a "drawstring elastic". In other nonlimiting examples, the layers of the elastic laminate may be joined at zero applied strain and subsequently activated.

In alternative embodiments, the waist feature may be inelastic. In such configurations, the waist feature may provide additional anchoring about the waist of the wearer.

In elastic or inelastic embodiments, the waist feature 50 may comprise an adjustment strip 40 as described in more detail below. In such embodiments, the adjustment strip is joined to outer cover materials at one or both ends of the adjustment strip, to permit movement of the strip in other areas. The adjustment strip 40 may be laterally-extending, meaning that the adjustment strip extends more in lateral dimension than in the longitudinal dimension.

Additionally, or alternatively, the article may comprise a leg gasketing system 52, portions of which may be formed by the outer cover and/or the absorbent insert. The outer cover may include one or more elastic elements, such as films or elastic strands, extending through all or a portion of the leg opening 51 to form a leg band portion 56. The elastic elements may be laminated with one or more nonwoven layers and/or one or more textile layers. The elastic elements may be laminated between the first and second materials 24, 26 described above. The elastic elements may be joined to the outer cover in a drawstring elastic configuration. As described with respect to the waist feature, the leg band portion may include rugosities as result of the lamination process. In other nonlimiting examples, the leg band portion may be a zero strain, activated laminate.

In various embodiments, the leg band comprises an adjustment strip 40. In such embodiments, the adjustment strip is joined to outer cover materials at one or both ends of the adjustment strip, to permit movement of the strip in other areas. The adjustment strip in the leg band is longitudinally-extending, meaning it extends more in the longitudinal dimension in the lateral dimension.

The waist features 50 and/or leg band portions 56 may be disposed along the edge of the outer cover 20, and in some circumstances, it may be desired to have elasticized waist and leg band portions situated along substantially the entire length of the leg and/or waist openings so as substantially or completely encircle the wearer's legs and/or waist while outer cover 20 is worn. The gathered material within rugosities can serve to accommodate stretching of waist feature and leg band portions. This arrangement not only may provide for better fit about the wearer's legs, but also may enable the outer cover 20, when formed of appropriately sized and shaped material, to form a pouch-like structure 53 in the crotch region 16 (see Fig. 1) when worn, which may serve to provide space within the outer cover 20 to accommodate the insert 30 and help hold it in place within outer cover 20, in a substantially laterally centered position within the crotch region 16. Alternatively, or additionally, the elastic strands or strips in waist features 50 and leg band portions 56 may be affixed within the outer cover 20 only at or near their respective ends, e.g., within a pouch, tube or envelope structure formed of outer cover material - referred to herein as a "drawstring elastic". This will allow the elastic material and associated outer cover material to stretch and move freely and independently of each other, which may promote fit and comfort. A snug fit about the wearer's legs provided by such elasticized leg band portions may serve to enhance containment capability of the wearable absorbent article. The outer cover 20 may also include anchoring supplements, bands or systems thereof as described in more detail in U.S. Patent No. 8,932,273.

### Adjustment Mechanisms in the Outer Cover

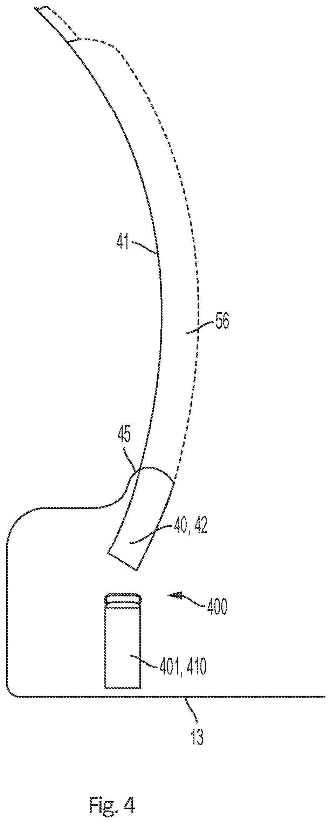

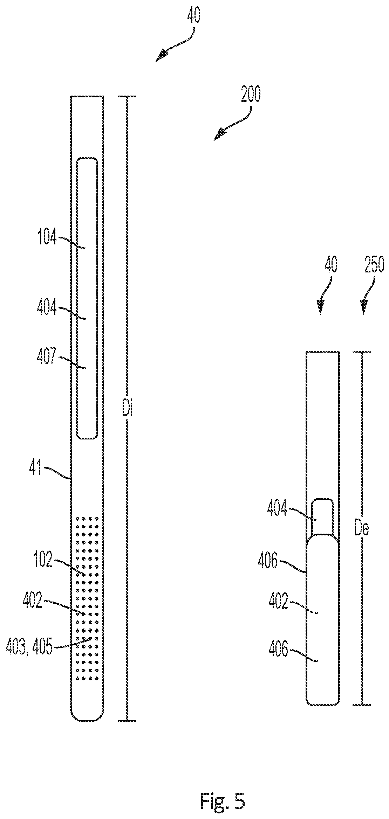

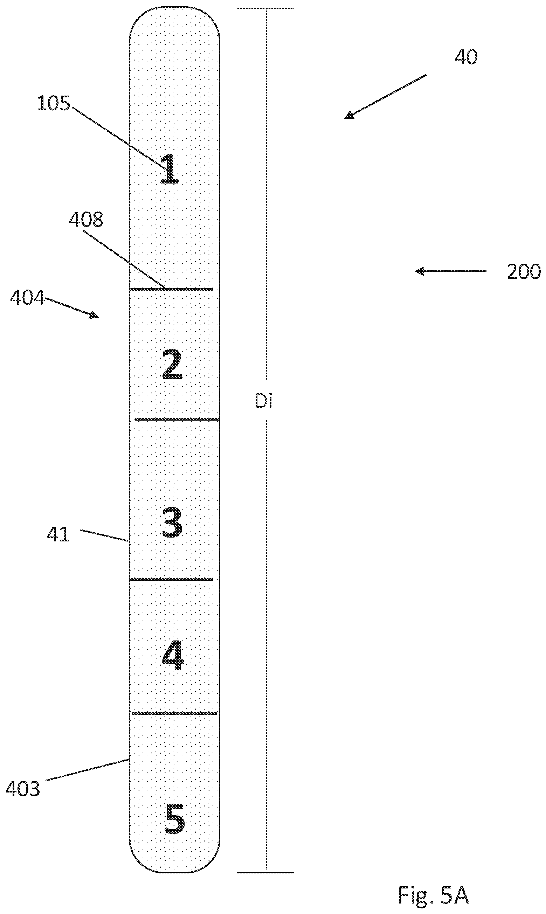

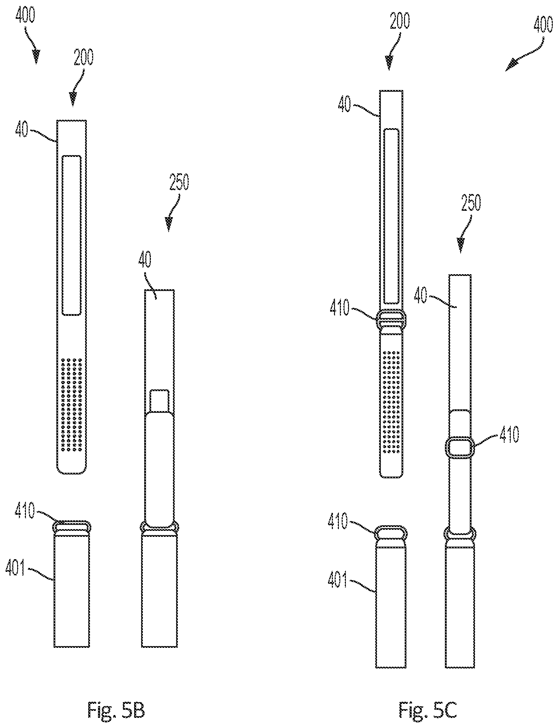

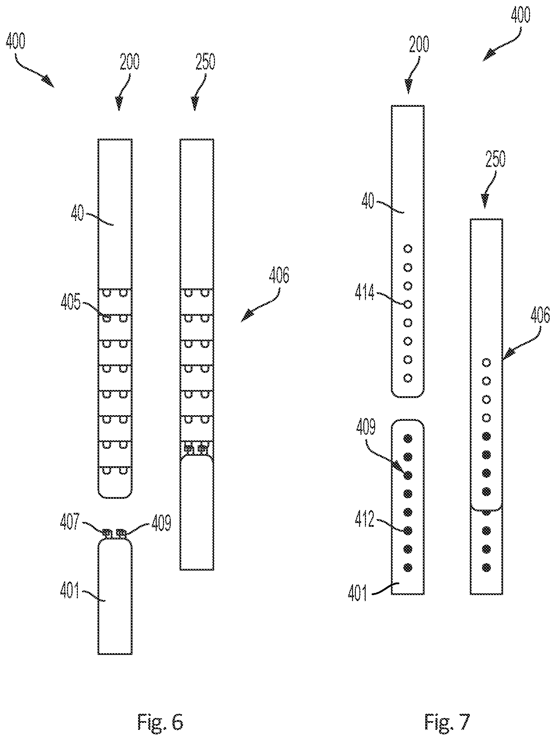

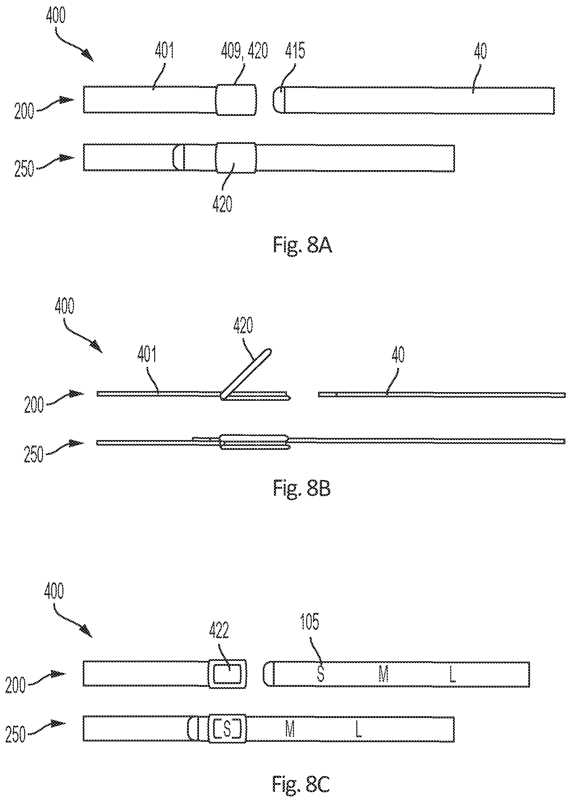

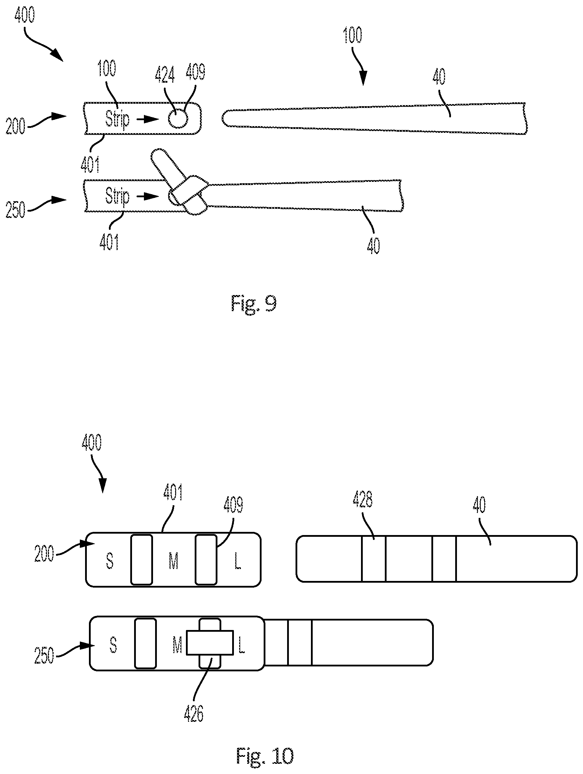

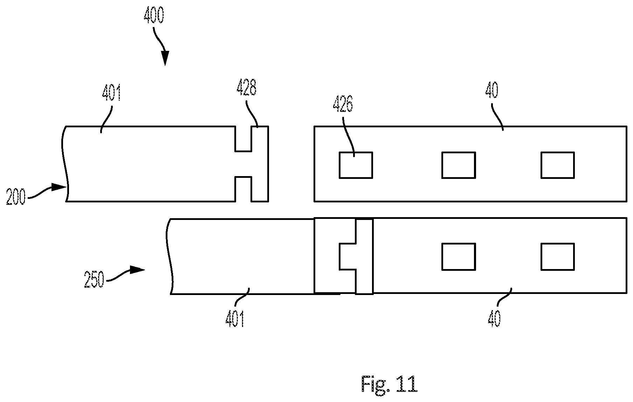

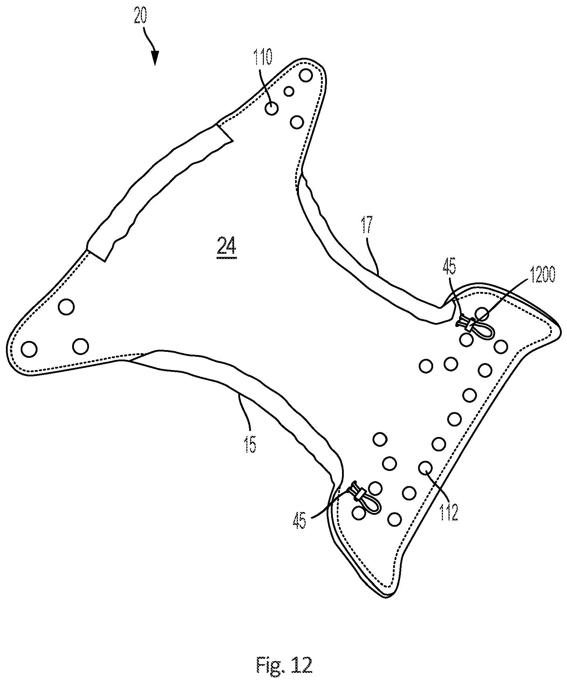

As shown in Fig. 4, the outer cover 20 comprises an adjustment system 400 comprising one or more adjustment strips 40. The adjustment system 400 is selected from the group consisting of buckle and/or clip mechanisms (see Figs. 8A-8D), snap mechanisms, lock and key mechanisms (see Fig. 11) such as twist lock mechanisms (see Fig. 10), tying mechanisms (see Fig. 9), hook and loop mechanisms (see Figs. 5-6), cord toggle (also known as cord lock or cord stop) mechanisms (see Figs. 12-13), male and female mechanisms (see Fig. 7) and combinations thereof. Elements of the adjustment system (e.g., buckles, hooks, loops, snaps locks, clips, cord toggles) may be any suitable shape or size. The adjustment system 400 comprises multiple engagement points 406. At each engagement point 406, the adjustment strip may be joined to the engaging mechanism to facilitate a change in dimension and/or tension in the adjustment strip 40.

As shown in Fig. 2, an adjustment strip 40 is disposed in a leg gasketing system 52. The adjustment strip 40 is a longitudinally-extending adjustment strip 42, meaning it extends more in the longitudinal direction than in the lateral direction. For purposes of longitudinally-extending, the longitudinal direction includes directions within 40 degrees of the maximum linear dimension in the x-y plane of the outer cover. In nonlimiting examples, an outboard edge 41 of the longitudinally-extending adjustment strip 42 may be disposed a maximum distance of about 20 mm or less, or about 15 mm or less, or about 10 mm or less, or about 5 mm or less, or from about 0 mm to about 20 mm, or from about 2 mm to about 10 mm from the first longitudinal edge 15 or the second longitudinal edge 17, reciting for each range every 1 mm increment therein.

For purposes of laterally-extending, the lateral direction includes directions within 40 degrees of the direction perpendicular to the maximum linear dimension in the x-y plane of the outer cover.

The adjustment strip is preferably an elastic element. However, it is also contemplated that the adjustment strip may be inelastic.

The adjustment strip may be accessed through a single opening 45 in the outer cover 20, or through multiple openings 45 in the outer cover 20. The opening(s) 45 may be disposed at or near an end of the leg band 56 or waist feature 50 or some other portion of said components. The opening(s) 45 may be covered by excess leg band material, excess waist feature material and/or a flap 47 as shown in Fig. 2. Any material covering the opening(s) 45 may be removably joined to a portion of the outer cover by any joining means, such as pressure-sensitive adhesive, hook and loop fasteners, and the like. The adjustment strip 40 may be enclosed within or between materials forming the external surfaces of the outer cover. In this way, the adjustment strip may be kept away from the wearer's skin during use, reducing the likelihood of abrasion and discomfort.

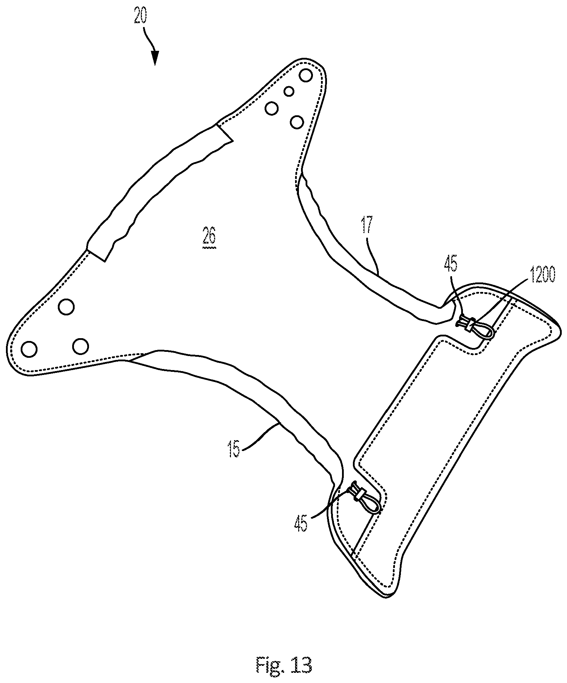

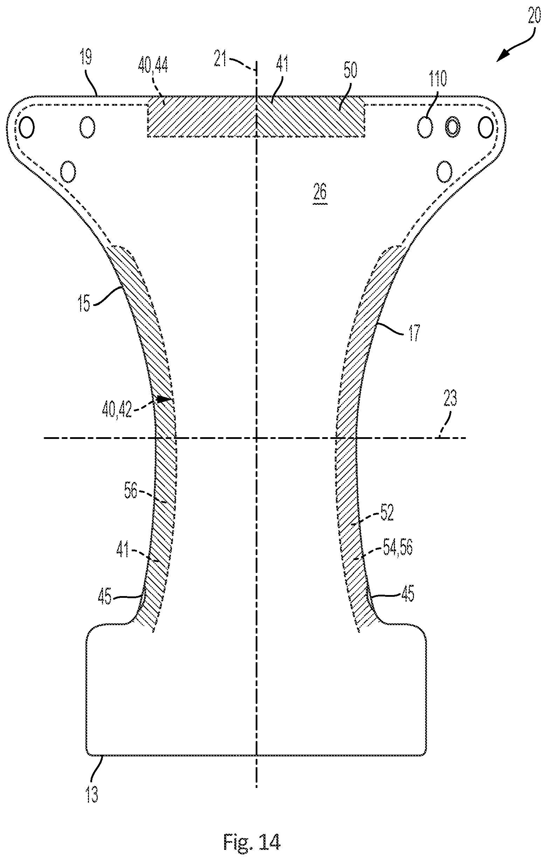

The adjustment strip may be accessed through opening(s) 45 disposed at or near the first longitudinal edge 15 and/or the second longitudinal edge 17 of the outer cover 20. The opening(s) may be disposed in the first material 24 (as seen in Fig. 12) and/or the second material 26 (as seen in Fig. 13). Disposition of the opening(s) 45 in the first material 24 may keep the adjustment mechanism easily accessible during wear, and may keep the mechanism away from the skin of the wearer. Disposition of the opening(s) 45 in the second material 26 may protect the adjustment mechanism from inadvertent adjustment during wear, and may provide for an uninterrupted and more attractive garment-facing surface. The opening(s) may also be disposed at the junction of the first material 24 and second material 26, such as at a seam that joins the two materials together. As shown in Fig. 14, openings 45 are formed at the first longitudinal edge 15 and the second longitudinal edge 17 of the outer cover 20, where the first material 24 and the second material 26 meet. The openings allow the user or care giver to access the adjustment mechanism of the adjustment strip while the garment is being worn, but keeps the adjustment mechanism within the interior of the outer cover to prevent a baby or child from accessing the mechanism.

In various embodiments, a signal 100 may be provided to help identify the location of the adjustment strip. Signals 100 may be in the form of printing (including graphics and/or indicia such as letters, symbols, pictograms, diagrams, geometric figures, arrows, numbers, marks) embossing, tinting or color differentiation in material, windows, changes in opacity, tabs, removable materials, and combinations thereof. A signal may be visible from the wearer-facing side and/or from the garment-facing side of the outer cover. The signal may at least partially overlap the adjustment strip as indicated by signal 100a in Fig. 2. The signal may not overlap the adjustment strip as indicated by signal 100b in Fig. 2 The signal may comprise a removable material 100c which may be removed by the user. One benefit of the removable signal is that it can be large without ultimately being intrusive during use. The removable material may be attached by any suitable means, including adhesive, and may be disposable. The removable material may comprise a sticker. Additionally, or alternatively, the removable material may comprise a string loop that may be made of a textile material or plastic material. In various nonlimiting embodiments, the removable material comprises a color that differs from surrounding portions of the outer cover. In other nonlimiting examples, the signal remains on the outer cover and comprises a durable or semi-durable material (e.g., permanent ink).

Turning to Fig. 4, which illustrates a leg band and partial waist region, the adjustment strip 40 may extend through the opening 45 and/or be pulled through the opening 45 to facilitate changing the size of the outer cover. In the leg band 56, the adjustment strip 42 may facilitate changing the longitudinal length of the leg band in the relaxed (uncontracted) state, thus permitting a tighter fit about the leg when shortening the length and a looser fit when extending the length. Similarly, in a waist feature, the adjustment strip 44 may facilitate changing the lateral width of the waist feature in the relaxed (uncontracted) state.

The adjustment system 400 may comprise one or more secondary features 401 that are discrete from the adjustment strip. The secondary feature shown in Fig. 4 is in the form of a guide 410. Other forms are within the scope of the invention and are discussed in more detail below.

As shown in Fig. 5, the adjustment strip 40 comprises an unadjusted maximum dimension, Di. The unadjusted maximum dimension is which is its greatest dimension of the adjustment strip in an unadjusted configuration 200 (i.e., full length/width possible in the outer cover). As mentioned above, the adjustment strip may be enclosed between external surfaces of the outer cover. The adjustment strip may be enclosed for at least about 50%, or at least about 60%, or at least about 75%, or at least about 90%, or from about 50% to about 100%, or from about 60% to about 95% of its unadjusted maximum dimension, Di, in the unadjusted configuration 200.

In Fig. 5, the adjustment strip comprises a fastening component 402 and a receiving component 404 that is operatively engageable with the fastening component. Nonlimiting examples of engageable fastening and receiving components include tape tabs, hook and loop fastening components, and interlocking fasteners such as tabs & slots (Figs. 10-11), locking buckles/clips (Figs. 8A-8D), cord toggles (Figs. 12-13), buttons, hook and loop mechanisms (Fig. 6), snaps, male and female mechanisms (Fig. 7), and/or hermaphroditic fastening components. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. The fastening component and/or the receiving component may further include a release tape or other material, including folded material, that protects the component from insult prior to use.

The fastening component 402 comprises one or more fastening elements 403 which cause the component to join with another surface, such as the receiving component 404. In various embodiments, fastening elements comprise hooks 405. The hooks may be microscopic as illustrated in Figs. 5-5C or macroscopic as illustrated in Fig. 6. Receiving component 404 comprises material adapted to fastenably cooperate with fastening elements, such as a section or patch adapted to serve as cooperative loops 407 material as shown in Figs. 5-5C, to provide a hook-and-loop fastening system combination. In other nonlimiting examples, the loops 407 may be macroscopic as illustrated in Fig. 6.

The fastening and/or receiving components may be discrete from and joined to the adjustment strip or may be integral the adjustment strip. In nonlimiting examples, fastening components and receiving components may be formed on the same patch of material. For example, a fastening component may be integrally formed from a patch of loops material joined to the adjustment strip.

The fastening component 402 may be spaced apart from the receiving component 404, as shown in Fig. 5. In nonlimiting embodiments, the fastening component may comprise a first signal 102 and the receiving component may comprise a second signal 104 to aid in the identification of the components. The first signal may differ from the second signal by one of the group consisting of color, opacity, shape of signal, size of signal and combinations thereof.

Additionally, or alternatively, the receiving component may be longitudinally inboard and/or laterally inboard of the fastening component. For example, in a leg band, the receiving component may be longitudinally inboard of the fastening component, such that the adjustment strip may be folded toward the lateral axis to effectuate the adjustment. In a waist feature, the receiving component may be laterally inboard of the fastening component, such that the adjustment strip may be folded toward the longitudinal axis to effectuate the adjustment. Alternatively, the fastening component may at least partially overlap the receiving component 404 as shown in Fig. 5A, where the entire adjustment strip is configured to serve as a receiving component.

Returning to Fig. 5, the fastening component may be engageable with the receiving component to arrive at an adjusted configuration 250. In the adjusted configuration, the adjustment strip comprises an adjusted maximum dimension, De, that is less than the unadjusted maximum dimension Di. As shown in Fig. 5, multiple engagement points 406 are possible depending on the relative size of the fastening and receiving components.

As shown in Fig. 5A, the adjustment strip 40 may comprise one or more fold lines 408. The fold lines may comprise a lower basis weight than surrounding areas, or may comprise areas in which the adjustment strip has been compressed or embossed, allowing them the adjustment strip to be folded about them.

Additionally, or alternatively, the adjustment strip 40 may comprise indicia 105 to proper sizing. Such indicia may be in the form of letters, numbers, or combinations thereof.

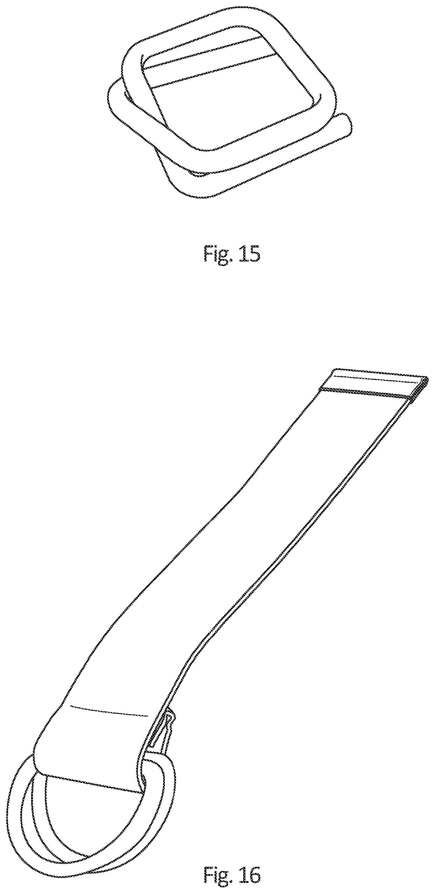

Turning to Fig. 5B, the outer cover may further comprise a secondary feature 401 discrete from the adjustment strip 40. In one form, the secondary feature 401 comprises a guide 410 through which the adjustment strip 40 can be threaded before the fastening component 402 is engaged with the receiving component 404. In this way, the adjustment strip 40 may be held more firmly in the desired location during use. The guide 310 may be in any form configured to engage the secondary feature 401 with the adjustment strip 40, including a single ring (Fig. 5B), a set of double rings (Fig. 15), or an x-buckle (Fig. 16). Additionally, or alternatively, a guide 410 may be disposed on the adjustment strip 40 itself (i.e., not on a discrete component of the outer cover) as shown in Fig. 5C. The guide also function to reverse the force on the strip by about 180 degrees, allowing for pulling in one direction to tighten the strip in the other direction.

While Figs. 5-5C illustrate microscopic hook and loops as fastening systems, it is also contemplated that macroscopic hooks and macroscopic loops may be used as fastening/receiving components on the adjustment strip. In other nonlimiting examples, male components 412 and female components 414 be used as the fastening/receiving components on the adjustment strip to resize the adjustment strip as described above. Male and female components are illustrated in Fig. 7.

Turning to Fig. 6, the adjustment system 400 may include a secondary feature 401 that is discrete from the adjustment strip 40 in an unadjusted configuration 200. The secondary feature 401 may comprise an engaging mechanism 409 which is operatively engageable with the adjustment strip 40 or portions thereof. The adjustment strip 40 is engaged with the engaging mechanism 409 to form the adjusted configuration 250.

As mentioned above, the adjustment system 400 may comprise multiple engagement points 406. The adjustment strip 40 may comprise a plurality of different engagement locations (e.g., a plurality of female openings 414 compatible with a male component 412 disposed on an engagement portion) and/or the engaging mechanism may comprise a plurality of different engagement locations (e.g., a plurality of female openings 414 compatible with a male component 412 disposed on the adjustment strip). For example, in Fig. 6, any hook-loop combination can serve as an engagement point. Likewise, in Fig. 7, any male-female combination can serve as an engagement point.

Referring to Fig. 6, in one form, the adjustment system comprises a hook-and-loop system. The hooks may be macroscopic and engage with macroscopic loops, as shown Fig. 6. Alternatively, the hooks and loops may be microscopic. The hooks may be provided on the adjustment strip and/or the secondary feature, serving as the engaging mechanism; and the loops may be provided on the adjustment strip and/or the secondary feature, serving as the engaging mechanism. The hooks and/or the loops may be integral with, or discrete and attached to, the relative component of the adjustment system.

Referring to Fig. 7, in another form, the adjustment system comprises a male-female mating system. The male and female components may be any suitable shape and size. Male components may comprise protrusions, for examples tabs or cylindrical protrusions. Male components may be provided on the adjustment strip and/or the secondary feature, serving as the engaging mechanism. Male components may be formed from, or discrete and attached to, the adjustment strip and/or the engaging mechanism. Female components may be provided on the adjustment strip and/or the secondary feature, serving as the engaging mechanism. The female components are shaped and sized to be compatible with the male components, and may be in the form of circular apertures or slots, for example.

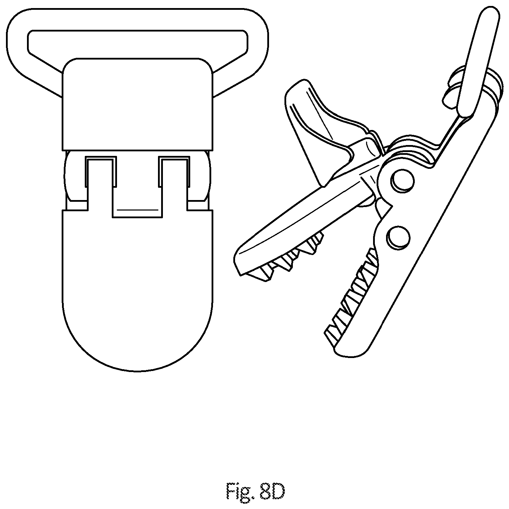

Turning to Figs. 8A-8D, in still another form, the adjustment system comprises a locking buckle 420 which fixes the adjustment strip in position relative to the secondary feature by compression, pins or other protrusions, magnetism and combinations thereof. The locking buckle may be disposed on the adjustment strip and/or the secondary feature, serving as the engaging mechanism. The locking buckle may comprise any suitable shape and size. As shown in Fig. 8B, the locking buckle is adapted to open to receive the adjustment strip or secondary feature, and to close to fix the adjustment strip into position. In some nonlimiting examples, the locking buckle comprises a window 422 through which the adjustment strip can be seen in the adjusted configuration as shown in Fig. 8C. In such examples, the window may allow the user to see indicia 105, which can help with proper sizing and adjustment. The locking buckle may be in the form of a locking alligator clip, as shown in Fig. 8D.

As shown in Fig. 9, in another nonlimiting example, the adjustment system comprises a tying mechanism, whereby the adjustment strip is coupled with the secondary feature through tying one to the other. In such nonlimiting examples, the engaging mechanism may comprise an aperture 424 through which the adjustment strip is threaded and subsequently tied. The adjustment strip may be tailored at its end to facilitate such threading. Additionally, or alternatively, the adjustment strip may comprise an aperture and the engaging mechanism may comprise material of the secondary feature that can be threaded through the adjustment strip.

Turning to Figs. 10 and 11, in still another form the adjustment system comprises a lock and key mechanism. As shown in Fig. 10, the lock and key mechanism may be in the form of a twist lock configuration. As shown in Fig. 11, the lock and key mechanism may be in the form of a static key configuration. The system comprises an aperture 426 and a protrusion 428 that fits within the aperture. The protrusion may twist to ensure it remains fixed in the adjusted configuration. The aperture may be disposed on the adjustment strip and/or on the secondary feature, serving as the engaging mechanism. The protrusion may be disposed on the adjustment strip and/or the secondary feature, serving as the engaging mechanism. The aperture and protrusion each may be any suitable shape and size.

Referring to Figs. 12 and 13, the adjustment system may comprise a cord toggle 1200 (also called a cord lock) system. An exemplary cord toggle system is disclosed in U.S. Patent No. 44,622,723, which is incorporated herein by reference. The cord toggle 1200 may generally be comprised of a barrel, a toggle that fits at least partially within the barrel, and a spring. The barrel and toggle may each comprise a hole that line up with each other when the toggle is plunged into the barrel, compressing the spring. As much of the adjustment strip may inserted into the hole in the barrel and toggle depending on the length of the adjustment strip desired. When tension on the spring is released, the toggle raises in the barrel, and the holes in the toggle and barrel are no longer aligned, causing the adjustment strip to be locked in place within the cord toggle mechanism.

In any of the foregoing forms, the adjustment strip, the secondary feature or other portions of the outer cover may comprise one or more signals 100, including indicia 105 to facilitate location of the adjustment strip, sizing, positioning of the adjustment system components and combinations thereof. The signal 100 may be provided on the secondary feature 401 as shown for example in Fig. 9. Additionally, or alternatively, a tab 415 may be provided on the adjustment strip and/or on the secondary feature in any of the foregoing embodiments, as illustrated for example in Figs. 8A-8C.

The secondary feature 401, and therefore the engaging mechanism 409, may be disposed proximate (i.e., about 20 mm or less) to an edge of the outer cover. When the adjustment strip is disposed in the leg band, the secondary feature may be disposed proximate to the front lateral end or proximate to the rear lateral end. When the adjustment strip is disposed in the waist feature, the secondary feature may be disposed proximate to the first or second longitudinal edge.

The article may comprise two or more adjustment strips, each having any of the features described above. For instance, the outer cover may comprise an adjustment strip in the leg band proximate to the first longitudinal edge and a second adjustment strip in the leg band proximate to the second longitudinal edge. Additionally, or alternatively, the outer cover may comprise an adjustment strip in the leg band proximate to one of the first or second longitudinal edges and a second adjustment strip in a waist feature. Where multiple adjustment strips are present in an outer cover, each adjustment strip may be independently adjusted and may differ by one of the group comprising: size, shape, material, degree of elasticity, type of fastening/receiving components, presence or absence of fastening/receiving components, type of adjustment system, color and combinations thereof.

### Ears and Fastening Systems

Returning to Figs. 1-2, the outer cover may include ears 70 in one or both of the front and rear regions. The ears may include fastening components 110 such that the ears can be secured to the opposing region (e.g., rear ears may include fastening components that may engage with the front region). The outer cover may include receiving components 112 to operatively engage with fastening components 110. Nonlimiting examples of engageable fastening and receiving components include tape tabs, hook and loop fastening components, interlocking fasteners such as tabs & slots, buckles, buttons, snaps, and/or hermaphroditic fastening components. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. A fastening component may be discrete from and joined to the outer cover or may be integral with the outer cover. The receiving component may be discrete from and joined to the outer cover or may be integral with the outer cover. The fastening component and/or the receiving component may further include a release tape or other material, including folded material, that protects the component from insult prior to use. In nonlimiting examples, a fastening component 110 is disposed on a wearer-facing surface 9 of the outer cover and a receiving component 112 is disposed on a garment-facing surface 8. In such nonlimiting examples, the outer cover may comprise two or more fastening systems having distinct fastening locations, which deconcentrates lateral tensile focuses and reduces the tendency of the front portion of the article to pivot around the single fastening location. Suitable dual fastening systems are disclosed for example in U.S. Pat. App. Nos. 16/684,230 and 62/975,836.

The outer cover may also comprise one or more insert components of an insert-to-outer cover fastening system. The outer cover may comprise fastening components capable of operatively engaging with an insert receiving component disposed on the absorbent insert, and/or may comprise receiving component(s) 112I capable of operatively engaging with an insert fastening component 110I disposed on the absorbent insert as shown in Figs. 2-3. The insert fastening and receiving components may comprise any of the exemplary components disclosed. The insert fastening and/or receiving components may be disposed on an interior surface of the outer cover, such that the absorbent insert is placed within a pocket of the outer cover. The insert fastening and/or receiving components may be disposed on an exterior surface, specifically the wearer-facing surface 9 of the outer cover. The fastening and/or receiving components may be discrete from and joined to the outer cover or absorbent insert or may be integral with one or both as is disclosed in U.S. Pat. App. Nos. 62/975,836.

### Absorbent Insert

Returning to Fig. 3, the absorbent insert 30 may be designed to contain and/or absorb body exudates, and may be made of pliable materials as will be described further below. The insert 30 includes a forward region 38 and a rearward region 39, a first lateral end 31 and a second lateral end 33, a first longitudinal edge 36, a second longitudinal edge 37, a lateral axis 60 and a longitudinal axis 62. The insert 30 comprises a length L from the outboard-most portion of front edge 31 to the outboard-most portion of the rear edge 33. The lateral axis equally divides the length Li. In nonlimiting examples, the insert length L is less than the length of the outer cover Lc.

The longitudinal edges 36, 37 may be generally parallel to the longitudinal axis. Alternatively, the longitudinal edges 36, 37 may be curved, such as in an hour-glass configuration. Thus, the width of the insert, Wi, may vary. In nonlimiting examples, the width of the insert, Wi, is less than the width of the outer cover, Wcv, at one or more longitudinal positions. The width of the insert, Wi, may be less than the width of the outer cover, Wcv, throughout the length of the insert.

The insert also may include one or more grasp structures 63. The grasp structures may be provided to enable the user to quickly and easily grasp the insert, handle the insert during application and/or properly place the insert. In certain embodiments, the grasp structure may have a different tactile feel than surrounding or adjacent areas to distinguish the area and ease the user's identification of the grasp structures.

The absorbent insert may be made of washable material such as cloth, or of a material suitable for composting, such as cellulose. In other embodiments, the absorbent insert may be disposable an made from materials commonly found in disposable absorbent articles.

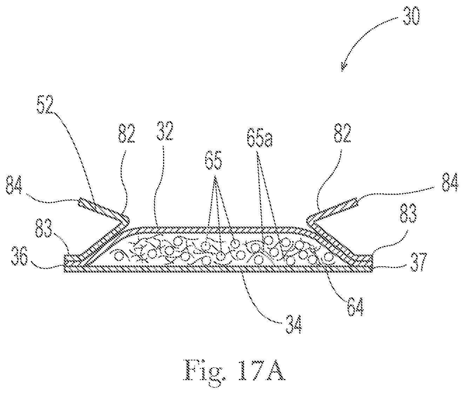

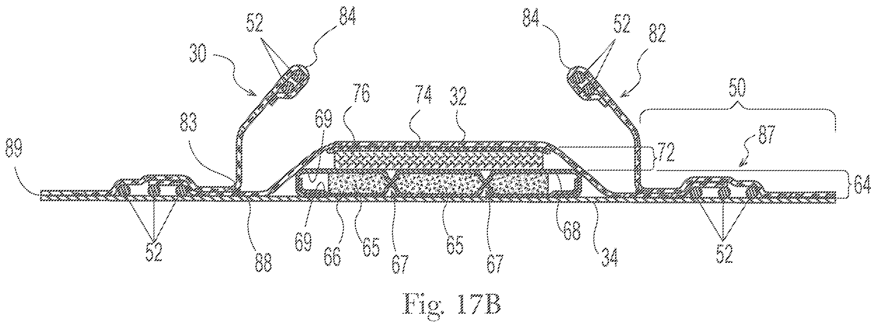

As shown in Figs. 17A-17B, the insert 30 may have a topsheet 32 and a backsheet 34. The topsheet and backsheet may be joined together. An absorbent core 64 may be disposed between the topsheet and the backsheet. As shown in Figs. 17A and 17B, the insert 30 may further include cuffs 80 affixed along the longitudinal sides 36, 37. Nonlimiting examples of absorbent inserts and details of their features are disclosed in U.S. Pat. Nos. 8,546,64 and 9,011,402.

### Topsheet

The topsheet 32 is generally a portion of the absorbent article 10 that may be positioned at least in partial contact or close proximity to a wearer. Suitable topsheets 32 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet 32 is generally supple, soft feeling, and non-irritating to a wearer's skin. Generally, at least a portion of the topsheet 32 is liquid pervious, permitting liquid to readily penetrate through the thickness of the topsheet 32. The topsheet 32 may comprise one or more apertures.

Any portion of the topsheet 32 may be coated with a lotion, antibacterial, or skin care composition as is known in the art. The topsheet 32 may be fully or partially elasticized or may be foreshortened so as to provide a void space between the topsheet 32 and the core 64.

Topsheet 32, backsheet 34 or any portion of the topsheet or backsheet may be embossed and/or matte finished to provide a more cloth-like appearance.

### Backsheet

Backsheet 34 is generally the outer liner portion of insert 30 forming the garment-facing surface 64 thereof, and prevents the exudates absorbed and contained within insert 30 from wicking through and soiling the outer cover. The backsheet 34 may comprise one or more nonwovens, films, elastomeric films, foams, strands, or combinations of these or other suitable materials with nonwovens or synthetic films. In nonlimiting examples, the backsheet is a laminate of an elastomeric material, such as a film, and a nonwoven.

In various embodiments, the backsheet 34 is substantially water-impermeable. Suitable backsheet 34 materials include nonwovens and/or films. Other suitable backsheet 34 materials may include breathable materials that permit vapors to escape from the absorbent article 10 while still preventing exudates from passing through the backsheet 34. Exemplary breathable materials may include materials such as woven webs, nonwoven webs, composite materials such as film-coated nonwoven webs, and microporous films.

In certain embodiments, the backsheet 34 may have a water vapor transmission rate (WVTR) of greater than about 2000 g/24h/m², greater than about 3000 g/24h/m², greater than about 5000 g/24h/m², greater than about 6000 g/24h/m², greater than about 7000 g/24h/m², greater than about 8000 g/24h/m², greater than about 9000 g/24h/m², greater than about 10000 g/24h/m², greater than about 11000 g/24h/m², greater than about 12000 g/24h/m², greater than about 15000 g/24h/m², measured according to WSP 70.5 (08) at 37.8 °C and 60% Relative Humidity. A higher WVTR may be desired in this particular application, since the insert backsheet 34 will not form the outer surface of the wearable article, as a conventional disposable diaper backsheet would, but rather, will be covered by the one or more layers of the outer cover material(s) - which themselves may act in some circumstances to reduce WVTR of the composite structure.

Other suitable materials and/or manufacturing techniques may be used to provide a suitable backsheet 34 including, but not limited to, surface treatments, particular film selections and processing, particular filament selections and processing.

Backsheet 34 may be joined to topsheet 32, absorbent core 64 or any other element of insert 30 by any suitable attachment mechanism known in the art. For example, the attachment mechanism may include a continuous line or layer of adhesive, a patterned layer of adhesive, or an array of separate lines, spirals, or spots of adhesive. One example of an attachment mechanism comprises an open pattern network of filaments of adhesive as disclosed in U.S. Patent No. 4,573,986. Other suitable attachment mechanisms include several lines of adhesive filaments which are swirled into a spiral pattern, as is illustrated by the apparatus and methods shown in U.S. Patents Nos. 3,911,173; 4,785,996; and 4,842,666. Adhesives which have been found to be satisfactory are manufactured by H. B. Fuller Company of St. Paul, Minnesota and marketed as HL-1620 and HL 1358-XZP. Alternatively, the attachment mechanism may comprise heat bonds, pressure bonds, ultrasonic bonds, dynamic mechanical bonds, stitches or any other suitable attachment mechanisms or combinations of these attachment mechanisms known in the art.

It will be appreciated that the outer cover described above can be constructed of materials and construction so as to bear and sustain a majority of the structural loading generally imposed upon a disposable diaper, by stretching and accommodation of the wearer's anatomical features and body movements, and by absorption, swelling and added weight resulting from the wearer's exudations of waste. Thus, lesser requirements for structural strength of an insert might be present with use of such an outer cover, as compared with strength required of inside components of a disposable diaper. Therefore, an article such as described herein may include a disposable absorbent insert manufactured from materials that are different from those ordinarily used in the manufacture of disposable diapers, such as petroleum-derived materials, e.g., polyethylene and polypropylene. For example, a disposable absorbent insert having one or more of a topsheet, backsheet, standing cuffs and/or other components formed of products of wood, cotton, flax (linen), hemp, bamboo, or other cellulose fibers (e.g., paper), in addition to the materials identified above, is contemplated. If resistance to aqueous liquid penetration or substantial liquid impermeability is desired, e.g., for a backsheet and/or standing cuffs, a material formed of ordinarily hydrophilic fibers such as paper may be coated or impregnated with a hydrophobic material, such as a skin-compatible oil or wax, to impart the desired resistance to aqueous liquid penetration. Each of the materials forming the insert may be selected so as to be dispersible in water or an aqueous solution, flushable, biodegradable and/or compostable (preferably to an agriculturally usable humus or soil amendment).

### Absorbent Core

Turning to Fig. 17A, the insert 30 may have an absorbent core 64 disposed within the envelope-like structure formed by the topsheet 32 and backsheet 34. The absorbent core 64 may comprise materials suitable for absorbency and for washing and reuse of the insert. In nonlimiting examples, the insert may be formed of cotton, fleece, flannel, felt, nylon, polyester, terry cloth and combinations thereof.

Alternatively, the absorbent core 64 may comprise a wide variety of liquid-absorbent materials 65 commonly used in disposable diapers and other absorbent articles. Examples of suitable absorbent materials include comminuted wood pulp, which is generally referred to as air felt creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials (AGM); or any other known absorbent material or combinations of materials. The absorbent core may have an absorbent capacity of at least about 120 g, or at least about 150 g, or at least about 200 g, or from about 120 to about 300 g, reciting for said range every 10 increment therein, as determined by the Core Capacity Test Method herein.

In some embodiments, the core is void of superabsorbent polymers and/or absorbent gelling materials. In other embodiments, at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. It should be understood that an immaterial amount of cellulosic material does not materially affect at least one of the thinness, flexibility, and absorbency of the portion of the absorbent core that is substantially cellulose free. Among other benefits, it is believed that when at least a portion of the absorbent core is substantially cellulose free, this portion of the absorbent core is significantly thinner and more flexible than a similar absorbent core that includes more than 10% by weight of cellulosic fibers. The amount of absorbent material, such as absorbent particulate polymer material 65a present in the absorbent core may vary, but in certain embodiments, is present in the absorbent core in an amount greater than about 80%, or greater than about 85%, or greater than about 90%, or greater than about 95% by weight of the core. In nonlimiting examples, a thermoplastic material, such as a thermoplastic adhesive composition 66, may be used to immobilize superabsorbent particles on a substrate (e.g., the topsheet, backsheet or core wrap).

In some embodiments, as shown in Fig. 17B, the absorbent core may comprise one or more channels 67, wherein said channels are substantially free of absorbent particulate polymer material. The channels 67 may extend longitudinally or laterally. The absorbent core may further comprise two or more channels. The channels may be straight, curvilinear, angled or any workable combination thereof. In nonlimiting examples, two channels are symmetrically disposed about the longitudinal axis.

The absorbent core 64 may include a core wrap 68, comprising one or more substrates 69 to encloses the absorbent material 65. Where channels are present, the core wrap may be bonded within one or more channels, thereby providing permanent channels which maintain their channel structure in the wet state.

The absorbent core 64 may be manufactured in a wide variety of sizes and shapes (*e.g.,* rectangular, hourglass, "T"-shaped, etc.). The configuration and construction of absorbent core 64 may also be varied (*e.g.,* the absorbent core(s) or other absorbent structure(s) may have varying caliper zones, hydrophilic gradient(s), a superabsorbent gradient(s), or lower average density and lower average basis weight acquisition zones; or may comprise one or more layers or structures).

### Acquisition-Distribution System

Referring to Figs. 17B, in some embodiments, an acquisition-distribution system (ADS) 72 is disposed between the topsheet 32 and the absorbent core 64. One function of the ADS is to quickly acquire one or more of the fluids and distribute them to the absorbent core in an efficient manner. The ADS may comprise one, two or more layers, which may form a unitary layer or may remain as discrete layers which may be attached to each other. The ADS 72 may include hydrophilic materials that provide significant wicking of bodily exudates. These materials may dewater the topsheet 32 and quickly move bodily exudates into the absorbent core 64. The ADS 72 may comprise one or more nonwoven materials, foams, cellulosic materials, cross-linked cellulosic materials, air laid cellulosic nonwoven materials, spunlace materials, or combinations thereof, for example. In some instances, portions of the ADS 72 may extend through portions of the topsheet 32, portions of the topsheet 32 may extend through portions of the ADS 72, and/or the topsheet 32 may be nested with the ADS 72. Typically, an ADS 72 may have a width and length that are smaller than the width and length of the topsheet 32. The ADS may have one or more channels as described above with reference to the absorbent core 64. The channels in the ADS may align or not align with channels 67 in the absorbent core 64. In an example, a first acquisition material 74 may comprise a nonwoven material and as second acquisition material 76 may comprise a cross-linked cellulosic material.

### Cuff Structures

As noted above, the article 10 may comprise a leg gasketing system 50, portions of which may be formed by the insert 30. Returning to Figs. 17A-17B, the insert may comprise a pair of longitudinal standing cuffs 82, also referred to as barrier leg cuffs. Each standing leg cuff may be formed by a piece of material which is bonded to the absorbent insert so it may extend upwards from a wearer-facing surface and provide improved containment of fluids and other body exudates approximately at the junction of the torso and legs of the wearer. The standing leg cuffs are delimited by a proximal edge 83 joined directly or indirectly to the topsheet 32 and/or the backsheet 34 and a free terminal edge 84, which is intended to contact and form a seal with the wearer's skin. In some embodiments, the free terminal edge 84 comprises a folded edge. The standing leg cuffs 82 extend at least partially between the front edge 31 and the rear edge 33 on opposite sides of the longitudinal centerline 62 and are at least present in the crotch region.

The standing leg cuffs may be integral with the topsheet 32 or the backsheet 34 or may be a separate material joined to the topsheet and/or backsheet. Each standing leg cuff 82 may comprise one, two or more elastic elements close to the free terminal edge 84 to provide a better seal. The standing cuff may be formed of any of a variety of substrates such as plastic films and woven or nonwoven webs of natural fibers (*e.g.,* wood or cotton fibers), synthetic fibers (*e.g.,* polyester or polypropylene fibers), or a combination of natural and synthetic fibers. In certain embodiments, the standing cuffs may be formed of a substantially liquid impermeable web to contain and isolate liquid exudates from the outer cover, outer clothing and environment of the wearer and/or may be formed of a vapor permeable web for breathability of the insert and article.

Referring to Fig. 17B, the article 10 may comprise a dual gasketing system, which includes the standing cuffs 82 and gasketing cuffs 87. The gasketing cuffs 87 may be joined to the insert 30, more particularly to the topsheet and/or backsheet. The gasketing cuffs may be disposed outboard of the standing cuffs and may provide a better seal around the thighs of the wearer. A gasketing cuff 87 may comprises a proximal edge 88 and a free terminal edge 89. The free terminal edge 89 may comprise a folded edge. Each gasketing cuff may comprise one or more elastic elements, which may be sandwiched between other layers of material, such as the portions of material forming the attached proximal portions of the standing cuffs, topsheet, backsheet, separate gasketing cuff material, or combinations thereof. The gasketing cuff may be formed of any of a variety of substrates such as plastic films and woven or nonwoven webs of natural fibers (*e.g.,* wood or cotton fibers), synthetic fibers (*e.g*., polyester or polypropylene fibers), or a combination of natural and synthetic fibers. As noted with respect to standing cuffs 82, gasketing cuffs may likewise be formed of any suitable web materials but preferably are formed of web materials that are effectively liquid impermeable while being vapor permeable, so as to contain the wearer's liquid exudates within the insert while permitting the insert to "breathe" to avoid excess humidity within the insert (which may overhydrate the wearer's skin and promote conditions such as diaper rash). Suitable materials include nonwoven, films, elastic strands and combinations thereof.

In further embodiments, the leg gasketing system comprises standing leg cuffs that are integral with gasketing cuffs. Suitable leg gasketing systems which may be part of the absorbent article are disclosed in U.S. Pat. Nos. 8,939,957; 3, 860,003; 7,435,243; 8,062,279.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A diaper outer cover (20) comprising:
a front lateral end (13) and a rear lateral end (19);
a first longitudinal edge (15) and a second longitudinal edge (17); and
an adjustment system (400) comprising:
an adjustment strip (40) disposed in a leg gasketing system (52); and
multiple engagement points (406);
wherein the adjustment system (400) is selected from the group consisting of buckle mechanisms (420), snap mechanisms, lock and key mechanisms (426, 428), tying mechanisms (424), hook and loop mechanisms (405, 407), male and female mechanisms (412, 414) and combinations thereof;
wherein the adjustment strip (40) has an unadjusted maximum dimension, Di and comprises a fastening component (402) and a receiving component (404) operatively engageable with the fastening component (402),
wherein the unadjusted maximum dimension Di is reduced by engaging the fastening component (402) with the receiving component (404), such that an adjusted maximum dimension, De, is less than the unadjusted maximum dimension, Di..

2. The diaper outer cover (20)according to claim 1 further comprising an engaging mechanism (409) that is operatively engageable with the adjustment strip (40).

3. The diaper outer cover (20) any of the preceding claims, wherein, in an unadjusted configuration (200), the fastening component (402) and the receiving component (404) at least partially overlap.

4. The diaper outer cover (20) according to any of the preceding claims, wherein the adjustment strip (40) is elastic.

5. The diaper outer cover (20) according to any of the preceding claims, wherein the adjustment strip (40) is longitudinally-extending (42) and wherein the adjustment strip (40) is disposed within 20 mm of a first longitudinal edge (15) of the diaper outer cover.

6. The diaper outer cover (20) according to any of the preceding claims, wherein, in an unadjusted configuration, the adjustment strip (40) is enclosed by exterior surfaces (8, 9) for at least 50% of the unadjusted maximum dimension Di.

7. The diaper outer cover (20) according to any of the preceding claims wherein the adjustment system (400) comprises hook and loop fasteners (405, 407).

8. The diaper outer cover (20) according to any of the preceding claims wherein the adjustment strip (40) comprises one or more fold lines (408).

9. The diaper outer cover (20) according to any of the preceding claims, wherein the adjustment (400) system comprises a locking buckle (420) capable of securing the adjustment strip (40) at the engagement points (406).

10. The diaper outer cover (20) according to any of the preceding claims, wherein the adjustment system (400) comprises one or more female receiving components and one or more male fasteners, wherein the male fasteners are operatively engageable with the female receiving components.

11. The diaper outer cover (20) according to any of the preceding claims further comprising a signal (100) identifying the adjustment strip (40), wherein the signal (100) includes printing, embossing, tinting and combinations thereof.

12. The diaper outer cover (20) according to claim 11 wherein the signal (100) comprises a removable material (100c).

13. The diaper outer cover (20) according to any of the preceding claims, wherein the adjustment strip (40) comprises sizing indicia (105).

## Patentansprüche

1. Windelaußenmantel (20), umfassend:
ein vorderseitiges seitliches Ende (13) und ein rückseitiges seitliches Ende (19);
eine erste Längskante (15) und eine zweite Längskante (17); und
ein Einstellsystem (400), umfassend:
einen Einstellstreifen (40), der in einem Beindichtungssystem (52) angeordnet ist; und
mehrere Eingriffspunkte (406);
wobei das Einstellsystem (400) ausgewählt ist aus der Gruppe, bestehend aus Schnallenmechanismen (420), Schnappverschlussmechanismen, Schloss- und Schlüsselmechanismen (426, 428), Bindemechanismen (424), Haken- und Schlaufenmechanismen (405, 407), männlichen und weiblichen Mechanismen (412, 414) und Kombinationen davon;
wobei der Einstellstreifen (40) eine nicht eingestellte maximale Abmessung, Di, aufweist und eine Befestigungskomponente (402) und eine Aufnahmekomponente (404) umfasst, die mit der Befestigungskomponente (402) wirksam in Eingriff gebracht werden kann,
wobei die nicht eingestellte maximale Abmessung Di durch das Ineingriffnehmen der Befestigungskomponente (402) mit der Aufnahmekomponente (404) derart reduziert wird, dass eine eingestellte maximale Abmessung, De, geringer als die nicht eingestellte maximale Abmessung, Di, ist..

2. Windelaußenmantel (20) nach Anspruch 1, ferner umfassend einen Eingriffsmechanismus (409), der mit dem Einstellstreifen (40) wirksam in Eingriff gebracht werden kann.

3. Windelaußenmantel (20) nach einem der vorstehenden Ansprüche, wobei, in einer nicht eingestellten Konfiguration (200), die Befestigungskomponente (402) und die Aufnahmekomponente (404) sich wenigstens teilweise überlappen.

4. Windelaußenmantel (20) nach einem der vorstehenden Ansprüche, wobei der Einstellstreifen (40) elastisch ist.

5. Windelaußenmantel (20) nach einem der vorstehenden Ansprüche, wobei der Einstellstreifen (40) sich längs erstreckend (42) ist und wobei der Einstellstreifen (40) innerhalb von 20 mm einer ersten Längskante (15) des Windelaußenmantels angeordnet ist.

6. Windelaußenmantel (20) nach einem der vorstehenden Ansprüche, wobei, in einer nicht eingestellten Konfiguration, der Einstellstreifen (40) durch Außenoberflächen (8, 9) für wenigstens 50 % der nicht eingestellten maximalen Abmessung Di umschlossen ist.

7. Windelaußenmantel (20) nach einem der vorstehenden Ansprüche, wobei das Einstellsystem (400) Haken- und Schlaufenbefestigungselemente (405, 407) umfasst.

8. Windelaußenmantel (20) nach einem der vorstehenden Ansprüche, wobei der Einstellstreifen (40) eine oder mehrere Faltenlinien (408) umfasst.

9. Windelaußenmantel (20) nach einem der vorstehenden Ansprüche, wobei das Einstellsystem (400) eine Verriegelungsschnalle (420) umfasst, die in der Lage ist, den Einstellstreifen (40) an den Eingriffspunkten (406) zu sichern.

10. Windelaußenmantel (20) nach einem der vorstehenden Ansprüche, wobei das Einstellsystem (400) eine oder mehrere weibliche Aufnahmekomponenten und einen oder mehrere männliche Befestigungselemente umfasst, wobei die männlichen Befestigungselemente mit den weiblichen Aufnahmekomponenten wirksam in Eingriff gebracht werden können.

11. Windelaußenmantel (20) nach einem der vorstehenden Ansprüche, ferner umfassend ein Signal (100), das den Einstellstreifen (40) identifiziert, wobei das Signal (100) ein Drucken, ein Prägen, ein Tönen und Kombinationen davon umfasst.

12. Windelaußenmantel (20) nach Anspruch 11, wobei das Signal (100) ein entfernbares Material (100c) umfasst.

13. Windelaußenmantel (20) nach einem der vorstehenden Ansprüche, wobei der Einstellstreifen (40) Größenmarkierungen (105) umfasst.

## Revendications

1. Couverture externe de couche (20) comprenant :
une extrémité latérale avant (13) et une extrémité latérale arrière (19) ;
un premier bord longitudinal (15) et un second bord longitudinal (17) ; et
un système d'ajustement (400) comprenant :
une bande d'ajustement (40) disposée dans un système d'étanchéité de jambe (52) ; et
de multiples points de mise en prise (406) ;
dans laquelle le système d'ajustement (400) est choisi dans le groupe constitué de mécanismes à boucle (420), mécanismes à encliquetage, mécanismes à verrou et clé (426, 428), mécanismes à nouage (424), mécanismes à crochets et boucles (405, 407), mécanismes mâles et femelles (412, 414) et combinaisons de ceux-ci ;
dans laquelle la bande d'ajustement (40) a une dimension maximale non ajustée, Di et comprend un composant d'attache (402) et un composant de réception (404) pouvant venir en prise fonctionnellement avec le composant d'attache (402),
dans laquelle la dimension maximale non ajustée Di est réduite par mise en prise du composant d'attache (402) avec le composant de réception (404), de telle sorte qu'une dimension maximale ajustée, De, est inférieure à la dimension maximale non ajustée, Di..

2. Couverture externe de couche (20) selon la revendication 1 comprenant en outre un mécanisme de mise en prise (409) qui peut venir en prise fonctionnellement avec la bande d'ajustement (40).

3. Couverture externe de couche (20) selon l'une quelconque des revendications précédentes, dans laquelle, dans une configuration non ajustée (200), le composant d'attache (402) et le composant de réception (404) se chevauchent au moins partiellement.

4. Couverture externe de couche (20) selon l'une quelconque des revendications précédentes, dans laquelle la bande d'ajustement (40) est élastique.

5. Couverture externe de couche (20) selon l'une quelconque des revendications précédentes, dans laquelle la bande d'ajustement (40) peut s'étendre longitudinalement (42) et dans laquelle la bande d'ajustement (40) est disposée à moins de 20 mm d'un premier bord longitudinal (15) de la couverture externe de couche.

6. Couverture externe de couche (20) selon l'une quelconque des revendications précédentes, dans laquelle, dans une configuration non ajustée, la bande d'ajustement (40) est enfermée par des surfaces extérieures (8, 9) sur au moins 50 % de la dimension maximale non ajustée Di.

7. Couverture externe de couche (20) selon l'une quelconque des revendications précédentes dans laquelle le système d'ajustement (400) comprend des attaches à crochets et boucles (405, 407).

8. Couverture externe de couche (20) selon l'une quelconque des revendications précédentes dans laquelle la bande d'ajustement (40) comprend une ou plusieurs lignes de pliage (408).

9. Couverture externe de couche (20) selon l'une quelconque des revendications précédentes, dans laquelle le système d'ajustement (400) comprend une boucle de verrouillage (420) capable de fixer la bande d'ajustement (40) au niveau des points de mise en prise (406).

10. Couverture externe de couche (20) selon l'une quelconque des revendications précédentes, dans laquelle le système d'ajustement (400) comprend un ou plusieurs composants de réception femelles et une ou plusieurs attaches mâles, dans laquelle les attaches mâles peuvent venir en prise fonctionnellement avec les composants de réception femelles.

11. Couverture externe de couche (20) selon l'une quelconque des revendications précédentes comprenant en outre un signal (100) identifiant la bande d'ajustement (40), dans laquelle le signal (100) comporte une impression, un gaufrage, une teinture et des combinaisons de ceux-ci.

12. Couverture externe de couche (20) selon la revendication 11 dans laquelle le signal (100) comprend un matériau amovible (100c).

13. Couverture externe de couche (20) selon l'une quelconque des revendications précédentes, dans laquelle la bande d'ajustement (40) comprend des indications de taille (105).
